# EUROPÄISCHE PATENTANMELDUNG

(11) **EP 1 342 783 A1**
(43) Veröffentlichungstag der Anmeldung: **10.09.2003**
(21) Anmeldenummer: 02005186.8
(22) Anmeldetag: 08.03.2002
(51) Int. Cl.: C12N 15/19, C12N 15/63, C07K 14/52, A61K 38/19

(54) **Proteolyseresistenter aktiver VEGF**

(71) Anmelder: Eming, Sabine, A.,Dr. med., 50931 Köln (DE)
(72) Erfinder: Eming, Sabine, A.,Dr. med., 50931 Köln (DE)
(74) Vertreter: Meyers, Hans-Wilhelm, Dr.Dipl.-Chem.

(57) **Zusammenfassung**

Gegenstand der Erfindung ist Endothelial Growth Factor (VEGF), bei dem das Alanin an AS-Position 111 gegen Prolin ausgetauscht ist. Das Arginin an AS-Position 110 kann dabei gegen eine andere Aminosäure substituiert sein. Die Erfindung betrifft auch Derivate der erfindungsgemäßen VEGF, Nucleinsäuren, Expressionssysteme, Arzneimittel und die Verwendung der VEGF-Mutanten der Erfindung zur Behandlung chronischer Wunden.

## Beschreibung

Gegenstand der Erfindung ist Vascular Endothelial Growth Factor (VEGF), bei dem das Alanin an AS-Position 111 gegen Prolin ausgetauscht ist. Das Arginin an AS-Position 110 kann dabei gegen eine andere Aminosäure substituiert sein. Die Erfindung betrifft auch Derivate der erfindungsgemäßen VEGF, Nucleinsäuren, Expressionssysteme, Arzneimittel und die Verwendung der VEGF-Mutanten der Erfindung zur Behandlung chronischer Wunden.

Ein wichtiges Stadium der kutanen Wundheilung ist die Ausbildung eines Granulationsgewebes. Eng mit letzterem verbunden ist das Einwandern neugebildeter Gefäße (Neoangiogenese). Zahlreiche experimentelle und klinische Studien zeigen, dass chronische Wunden durch eine gestörte Angiogenese und somit verminderte Ausbildung eines Granulationsgewebes charakterisiert sind.

Es sind eine Vielzahl von Mediatoren bekannt, die die Angiogenese während der Wundheilung stimulieren. Zu diesen zählen zum einen solche Faktoren, die neben der Stimulation von Endothelzellen auch mesenchymale und/oder epidermale Zellen aktivieren (bFGF, aFGF, TGF-*a*, PDGF) und zum anderen sogenannte endothelzellspezifische Faktoren, deren Rezeptoren im wesentlichen auf Endothelzellen beschränkt sind (VEGF, Angiopoietin). Eine Vielzahl physiologischer und pathologischer Reaktionen unter Beteiligung der Blutgefäße korreliert mit einer erhöhten Expression von VEGF und seiner Rezeptoren, so dass VEGF eine zentrale Rolle in der Angiogenese der Haut zukommt. Erste Hinweise für die mögliche Bedeutung des VEGF bei Wundheilungsstörungen boten sich auf der Grundlage von Experimenten zur VEGF Expression bei diabetischen Mäusen (db/db Mäuse) (Frank *et al.* 1995). In diesem Modell konnte gezeigt werden, dass die Wundheilungsstörung mit einer verminderten VEGF Expression korreliert. Die Rolle von VEGF bei der Wundheilung konnte kürzlich durch ein weiteres transgenes Tiermodell (Fukumura *et al.,* 1998) und den Nachweis von VEGF im Wundsekret akuter humaner Wunden unterstützt werden (Nissen *et al*., 1998).

Weiterhin wurde gezeigt, dass die mRNA von VEGF und seinen Rezeptoren im Gewebe chronischer Wunden vermehrt exprimiert werden (Lauer *et al.,* 2000). Untersuchungen mittels SDS-PAGE zeigen jedoch einen Abbau des VEGF-Proteins im chronischen Wundmilieu, im Gegensatz zur akuten Wunde. Dieser Abbau führt zu einer signifikanten Einbuße der biologischen Aktivität und kann somit, trotz der erhöhten Expression der VEGF-Rezeptoren, einer defizienten Stimulation der Neoangiogenese im chronischen Wundmilieu zugrunde liegen. Wie oben erläutert, konnte gezeigt werden, dass Plasmin an der Spaltung von VEGF im chronischen Wundmilieu beteiligt ist (Lauer *et al.,* 2000).

Die Spaltung von VEGF₁₆₅ durch Plasmin führt zur Abtrennung der carboxylterminalen Domäne, die vom Exon 7 kodiert wird. Während die Bindungseigenschaften von VEGF an die VEGF-Rezeptoren Flt-1 und Flk-1/KDR über Exon 3 und 4 bestimmt werden, besitzt Exon 7 eine kritische Bedeutung in der Interaktion von VEGF mit Neuropilin-1 (Keyt *et al.* 1996). Neuropilin -1 ist ein 130 kDa Glykoprotein der Zelloberfläche. Erst vor kurzem wurde seine Rolle bei der Potenzierung des mitogenen Effekts von VEGF auf Endothelzellen beschrieben (Soker *et al.* 1998). Dabei scheint die Interaktion von Neuropilin-1 mit Flk-1/KDR bedeutsam, da die alleinige Bindung von VEGF an Neuropilin-1 keine Signalwirkung ausübt.

Plasmin gehört zur Klasse der Serinproteasen. Diese Enzyme sind in der Lage, Peptidbindungen zu spalten. Die Spaltung erfolgt über eine sogenannte katalytische Triade. Dabei spielen im katalytischen Zentrum insbesondere das namensgebende Serin, aber auch die Aminosäuren Histidin und Aspartat eine wesentliche Rolle, da über sie der Prozeß der Peptidspaltung erfolgt (Stryer 1987, S. 231 ff). Obwohl der Mechanismus der Bindungsspaltung bei allen Serinproteasen identisch ist, unterscheiden sie sich deutlich in ihrer Substratspezifität. So spaltet Plasmin, ebenso wie Trypsin, Peptidbindungen nach den basischen Aminosäuren Lysin und Arginin. Die Substratspezifität von Plasmin, die durch die Struktur des katalytischen Zentrums bestimmt wird, führt jedoch dazu, dass Plasmin nicht jede dieser Bindungen spalten kann. Die Katalyse der Peptidbindungsspaltung kann nur erfolgen, wenn die entsprechenden Proteinabschnitte in der Lage sind mit dem katalytischen Zentrum des Enzyms zu interagieren (Powers *et al*. 1993; Stryer 1987). Bisher ist keine eindeutige Konsensussequenz für eine Plasmin-Schnittstelle bekannt.

Der vorliegenden Erfindung liegt die Aufgabe zugrunde, verbesserte Mittel zur Heilung von chronischen Wunden bereitzustellen.

Überraschenderweise wird diese Aufgabe gelöst durch die erfindungsgemäße Bereitstellung von Vascular Endothelial Growth Factor (VEGF)-Varianten, wobei das Alanin an AS-Position 111 des Wildtyp VEGF gegen Prolin ausgetauscht ist. Gegenstand der Erfindung sind auch Mutanten (Varianten) des VEGF, bei denen zusätzlich das Arginin an AS-Position 110 gegen eine andere Aminosäure ausgetauscht ist.

Die erfindungsgemässen Mutanten von VEGF liegen vorzugsweise als eine der Splice-Varianten VEGF₁₂₁, VEGF₁₄₅, VEGF₁₆₅, VEGF₁₈₃, VEGF₁₈₉ oder VEGF₂₀₆.vor.

Die erfindungsgemäßen Mutanten von VEGF weisen nicht nur eine deutlich erhöhte Stabilität gegenüber Plasmin, sondern auch eine dem Wildtyp-VEGF vergleichbare Aktivität auf. Überraschenderweise weisen die erfindungsgemäßen VEGF-Varianten darüber hinaus eine deutlich erhöhte Stabilität in chronischen Wundflüssigkeiten auf.

Die Mutationen wurden an einer für die biologische Aktivität des VEGF-Moleküls kritischen Stelle durchgeführt. Somit war zu befürchten, dass eine Veränderung der Proteinstruktur in diesem Bereich die Aktivität von VEGF₁₆₅ negativ beeinflusst. Bei der Aminosäure Prolin, die erfindungsgemäß an Position 111 eingeführt wird, handelt es sich um eine zyklische α-Iminosäure.

Durch die zyklische Form des Pyrrolidin-Restes besitzt sie eine starre Konformation, die sich auch auf die Struktur der jeweiligen Proteine auswirkt. So fungiert Prolin z.B. als starker α-Helix-Brecher. Es ist daher in besonderem Masse überraschend, dass gerade durch den Ersatz des Alanins an Position 111 gegen Prolin eine Mutante von VEGF erzeugt wird, die stabil gegenüber der Protease Plasmin ist, stabil in chronischen Wundflüssigkeiten ist und zugleich noch eine dem Wildtyp-Protein entsprechende Aktivität aufweist.

Gegenstand der Erfindung sind insbesondere VEGF-Varianten der beiden Sequenzen Seq. No. 1 oder Seq. No. 2.

Gegenstand der Erfindung sind auch Varianten der oben aufgeführten VEGF-Mutanten, bei denen die Aminosäuresequenzen modifiziert oder derivatisiert sind oder Mutationen, Insertionen oder Deletionen enthalten. Insbesondere betrifft dies VEGF-Varianten, bei denen einzelne weiter Aminosäuren ausgetauscht sind und solche, die glykosyliert, amidiert, acetyliert, sulfatiert oder phosphoryliert sind. Vorzugsweise haben solche VEGF-Varianten eine dem Wildtyp-VEGF vergleichbare oder höhere Aktivität.

Die erfindungsgemäßen VEGF-Varianten können auch eine Signalsequenz aufweisen. Die Signalsequenz kann N-terminal an die Aminosäurekette der VEGF-Variante anschließen und die Sequenz 
Met Asn Phe Leu Ser Trp Ser Val His Trp Ser Leu Ala Leu
Leu Leu Tyr Leu His His Ala Lys Trp Ser Gln Ala
 aufweisen.

Gegenstand der Erfindung sind auch Nucleinsäuren, die für die oben benannten VEGF-Mutanten kodieren, und Vektoren zur Expression von VEGF, die solche Nucleinsäuren enthalten.

Gegenstand der Erfindung ist auch ein Arzneimittel, das die oben benannten Mutanten des VEGF enthält, sowie die Verwendung der VEGF-Mutanten zur Herstellung eines Arzneimittels zur Behandlung von chronischen Wunden, verursacht durch Gefäßveränderungen wie chronisch-venöse Insuffizienz (CVI), primäres/sekundäres Lymphödem, arterielle Verschlußkrankheit, metabolische Erkrankungen wie Diabetes mellitus, Gicht oder Dekubitus, chronisch entzündlichen Erkrankungen wie Pyoderma gangraenosum, Vaskulitis, perforierende Dermatosen wie Necrobiosis lipoidica diabeticorum und Granuloma annulare, hämatologische. Grunderkrankungen wie Gerinnungsstörungen, Sichelzellenanämie und Polycythemia vera, Tumoren wie primäre kutane Tumoren und exulzerierte Metastasen, sowie zur Plasmininhibition, zur Induktion der Neoangiogenese und/oder zur Inhibition der Matrixdegradierung.

Die topische Anwendung von Wachstumsfaktoren stellt in der Wundheilung ein neuartiges Therapiekonzept dar. Eine Verbesserung der Heilung chronischer Wunden konnte in einer Vielzahl von klinischen Studien mit der Anwendung von EGF, bFGF, PDWHF und PDGF beobachtet werden (Scharffetter-Kochanek *et al.* 2000). Es bleibt jedoch kritisch anzumerken, dass die Ergebnisse dieser Studien hinter den Erwartungen zurückgeblieben sind, die angesichts der guten Wirksamkeit dieser Mediatoren im Tiermodell bestanden haben (Lawrence *et al.* 1994). Eine wesentliche Erklärung für diese eingeschränkte Wirksamkeit der Wachstumsfaktoren ist sicherlich die erhöhte proteolytische Aktivität im chronischen Wundmilieu, die zur Degradation der topisch applizierten Fakturen führt. Somit wird deutlich, dass das lokale Wundmanagement durch die Applikation von Wachstumsfaktoren eine vielversprechende neue therapeutische Strategie darstellt. Jedoch ist es notwendig Strategien zu entwickeln, die die proteolytische Aktivität im chronischen Wundmilieu kontrollieren. Die Herstellung von Masterzytokinen mit erhöhter Stabilität im chronischen Wundmilieu stellt dabei sicherlich einen neuartigen Therapieansatz dar. Die erfindungsgemäßen VEGF-Mutanten eignen sich aufgrund ihrer hohen Stabilität in Wundflüssigkeit in besonderem Maße zur topischen Behandlung von chronischen Wunden.

### Ausführungsbeispiel:

### Mutagenese:

Mittels zielgerichteter Mutagenese wurden vier Mutanten hergestellt, indem gezielte Aminosäure-Austausche bei Arg₁₁₀ und Ala₁₁₁ vorgenommen wurden. Der cDNA, die für humanen VEGF₁₆₅ kodiert, wurde in den SV40 Replikations Expressionsvektor pcDNA 3.1 (Fa. Invitrogen, De Schelp, NL) kloniert unter Verwendung der Schnittstellen BamHI und EcoRI in der Cloning Site. Für die zielgerichtete *in vitro* Mutagenese wurde das Gene Editor™-System der Firma Promega (Mannheim) verwendet. Dieses basiert auf der Anlagerung von Oligonukleotiden, welche die entsprechenden Mutation tragen, an die Ausgangssequenz. Die Ausgangssequenz des VEGF₁₆₅ im Bereich der Mutationen ist:

106 107 108 109 110 111 112 113
GA CCA AAG AAA GAT AGA GCA AGA CAA G
Pro Lys Lys Asp Arg Ala Arg Gln

Zur Einführung der Mutationen wurden die folgenden "Missmatch"-Oligonucleotide als Primer verwendet:

Mutation 1 : MutAla:

GA CCA AAG AAA GAT GCC GCA AGA CAA G

Pro Lys Lys Asp Ala Ala Arg Gln

Mutation 2 : MutGln:

GA CCA AAG AAA GAT CAG GCA AGA CAA G

Pro Lys Lys Asp Gln Ala Arg Gln

Mutation 3 : Mut_{Pro}:

GA CCA AAG AAA GAT AGG CCA AGA CAA G

Pro Lys Lys Asp Arg Pro Arg Gln

Mutation 4 : MutLys-Pro:

GA CCA AAG AAA GAT AAG CCA AGA CAA G

Pro Lys Lys Asp Lys Pro Arg Gln

Die verwendeten Mutagenese-Primer werden jeweils mit den dadurch erhaltenen veränderten Aminosäuresequenzen aufgeführt. Kursiv markiert sind die Bereiche mit den gegenüber der Wildtypsequenz veränderten Basen oder Aminosäuren.

Bei Mutation 1 wurde das Arginin₁₁₀ gegen ein unpolares Alanin ausgetauscht. Bei Mutation 2 wurde an der selben Position ein polares, ungeladenes Glutamin eingeführt. Bei der Mutante 3 wurde nicht das basische Arginin₁₁₀, sondern das Alanin an Position 111 gegen ein Prolin ausgetauscht. Bei der Mutante 4 wurden zwei Aminosäuren ausgetauscht. Hier wurden anstelle von Arginin₁₁₀ und Alanin₁₁₁ Lysin und Prolin eingefügt. Nach der Durchführung der Mutagenese erfolgte die Verifizierung der Mutationen durch Sequenzanalyse. Die erhaltenen VEGF-Mutanten hatten bei Aminosäuren 109-112 folgende Sequenzen:
VEGF₁₆₅-Wildtyp: -Asp₁₀₉ Arg₁₁₀ Ala₁₁₁ Arg₁₁₂-
Mut_{Gln} : -Asp₁₀₉ Gln₁₁₀ Ala₁₁₁ Arg₁₁₂-
Mut_{Ala} : -Asp₁₀₉ Ala₁₁₀ Ala₁₁₁ Arg₁₁₂-
Mut_{Pro} : -Asp₁₀₉ Arg₁₁₀ Pro₁₁₁ Arg₁₁₂-
Mut_{Lys-Pro} : -Asp₁₀₉ Lys₁₁₀ Pro₁₁₁ Arg₁₁₂-

Die Mutanten Mut_{Pro} und Mut_{Lys-Pro} sind erfindungsgemäße Mutanten, während die Mut_{Gln} und Mut_{Ala} zu Vergleichszwecken hergestellt und untersucht werden. Die erhaltenen VEGF₁₆₅-Expressionsvektoren wurden in den weiteren Untersuchungen verwendet.

### Herstellung von rekombinantem VEGF₁₆₅ Protein

Die Expression von VEGF₁₆₅ Protein erfolgte in eukaryontischen COS-1-Zellen. Der verwendete Expressionsvektor pcDNA 3.1 enthält einen SV-40 Origin of Replication. Dieser dient zur Amplifikation des Vektors in Zellen, die ein Large T-Antigen des SV-40 Viruses exprimieren. Die verwendeten COS-1 Zellen besitzen ein entsprechendes im Genom integriertes Element, so dass es hier zu einer episomalen Replikation des Vektors kommt. Dadurch wird ohne die stabile Integration (Transformation) des Vektors ins Zellgenom eine Expression des Zielproteins VEGF für mehrere Tage erreicht. Die COS-1 Zellen wurden mit den bei der Mutagenese erhaltenen Expressionsplasmiden transfiziert. Dazu wurde das Superfect Transfection Reagent (QIAGEN, Hilden) nach den Vorschriften des Herstellers verwendet.

Wie eine Vielzahl von Wachstumsfaktoren besitzt auch VEGF₁₆₅ eine Heparin-Bindungsstelle, die am basischen C-Terminus lokalisiert ist. Die Bindung an Heparin wurde für die Aufreinigung des Proteins mittels Affinitätschromatographie genutzt (Mohanraj *et al.* 1995). Die Isolation der VEGF und VEGF-Varianten erfolgte durch die folgenden Schritte:

Die mit den Expressionsplasmiden transformierten COS-1-Zellen wurden in Serum-freiem DMEM (Dulbecco's modified-Eagle's Medium), enthaltend 10% fetales Kälberserum (FCS), 2 mM L-Glutamin, Penicillin (10 U/ml) und Streptomycin (10 µg/ml) und ITS Supplement (Sigma, Deisenhofen), kultiviert. Konditioniertes Medium (200 ml) wurde nach 48h gesammelt und für 4 Stunden mit 5 ml Heparin-Sepharose (Pharmacia, Freiburg) bei 4 °C inkubiert. Die Heparin-Sepharose wurde in eine Säule gepackt. Diese wurde bei einer Flußrate von 25 ml/h mit Kulturmedium beladen. Es wurden folgende Schritte durchgeführt:
A: Affinitätschromatographie mit Heparin-Sepharose
   1. Waschen: 0,1 M NaCl; 20 mM Tris/pH 7,2
   2. Waschen: 0,3 M NaCl; 20 mM Tris/pH 7,2
   3. Elution: 0,9 M NaCl; 20 mM Tris/pH 7,2
B: Analyse der erhaltenen Fraktionen durch Western-Blot Analyse
C: Entsalzung der VEGF-haltigen Fraktionen durch Gelfiltration
   Laufpuffer: 10 mM Tris/pH 7,2
D: Lyophilisierung der Lösung und Konzentrationsbestimmung durch ELISA

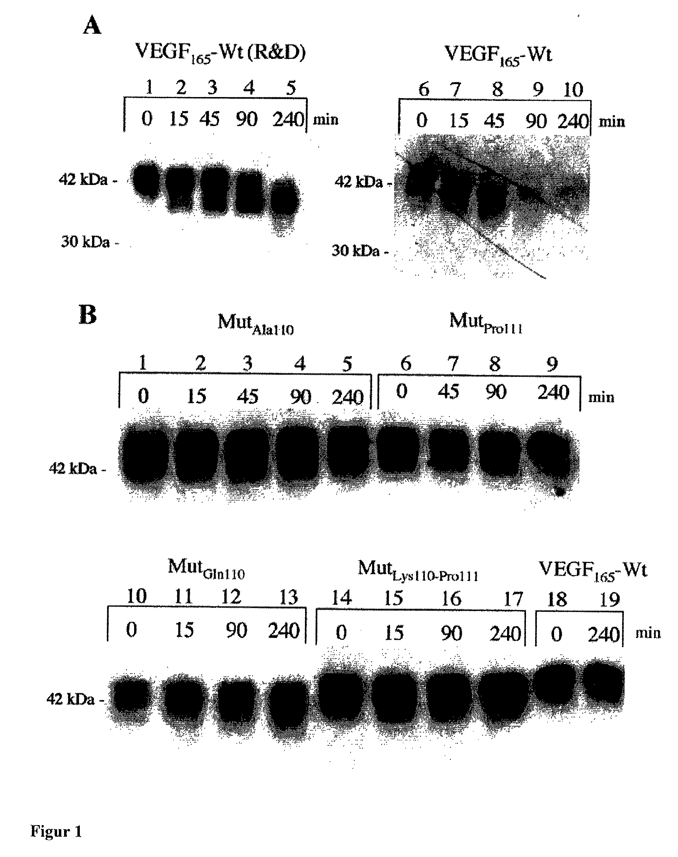

Das erhaltene VEGF wurde durch SDS-PAGE untersucht. Das aus COS-1-Zellen gewonnene VEGF-Protein unterscheidet sich in seinem Laufverhalten in der SDS-PAGE von dem verwendeten, kommerziell erhältlichen VEGF₁₆₅ Protein (Firma R&D Systems). Zusätzlich zu dem bei 42 kDA zu detektierende Signal (Figur 1, Bahn 6) ist noch eine weitere Bande mit einem einige kDa höheren Molekulargewicht zu erkennen. Grund für diese Doppelbande des in COS-1-Zellen exprimierten VEGF-Proteins ist eine veränderte Glykosylierung des Wachstumsfaktors. In COS-1-Zellen kommt es bei der Expression von VEGF zur Bildung zweier unterschiedlich glykosylierter Proteine. Eine Form (42 kDa) ist in ihrer Glykosylierung identisch mit dem bisher verwendeten rekombinanten VEGF₁₆₅, das in Insektenzellen mit einem Baculovirus-Expressionssystem erzeugt wurde (R&D Systems, Figur 1, Bahn 1). Es weist an der Aminosäure Asparagin bei Position 74 eine N-Glykosylierung auf (Gospodarowicz *et al.* 1989; Keck *et al.* 1989). Die zweite Bande bei höherem Molekulargewicht (45 kDa) entsteht auf Grund einer weiteren Glykosylierung des Proteins. Der Unterschied in der Glykosylierung ist für die Expression in COS-Zellen bekannt und hat keinen Effekt auf die biologische Aktivität des Wachstumsfaktors (R&D Systems).

### Charakterisierung der biochemischen und biologischen Eigenschaften der aufgereinigten VEGF₁₆₅ Proteine

### I. Analyse der Stabilität des VEGF₁₆₅-Proteins und seiner Mutationen:

### a) Inkubation in Plasmin

Die vier aufgereinigten mutierten VEGF-Proteine wurden zunächst auf ihre Stabilität gegenüber der Protease Plasmin untersucht. Es wurde untersucht, ob die vorgenommenen Mutationen zu einem verändertem Degradationsverhalten im Vergleich zu Wildtyp VEGF führen.

Figur 1 zeigt die Ergebnisse einer Inkubation des VEGF-Wildtyps und der VEGF-Mutanten mit Plasmin. Die Inkubation des in COS-1-Zellen synthetisierten VEGF-Wildtyps (A, Bahn 6-10) zeigt schon nach 15 Minuten eine Degradation des Wachstumsfaktors. Dabei ist die exakte Größenbestimmung der entstehenden Fragmente mittels SDS-PAGE schwierig, da sich die Signale mit den beiden Banden des unterschiedlich glykosylierten Proteins überlagern. Das Degradationsmuster ist jedoch dem des kommerziell erhältlichen VEGF₁₆₅ ähnlich (Figur 1A, Bahn 1 - 5). So kann nach 45 Minuten ein Fragment mit einem Molekulargewicht von 38 kDa detektiert werden. Dieses entspricht dem 110 Dimer-Fragment der weniger glykosylierten VEGF-Variante. Diese Ergebnisse zeigen deutlich, dass auch das VEGF-Protein, das in den COS-1-Zellen exprimiert wurde, unter den gewählten Bedingungen von Plasmin gespalten wird.

In Figur 1B (Bahn 1 - 17) sind die Ergebnisse der Inkubation mutierter Proteine zu sehen. Zunächst ist die Inkubation der Mutation Arginin zu Alanin dargestellt (Bahn 1 - 5). Zum Zeitpunkt Null der Inkubation sind wie beim Wildtyp zwei Banden für die unterschiedlich glykosylierten Varianten des VEGF-Proteins zu detektieren. Diese sind hier allerdings auf Grund der höheren Signalintensität nicht so eindeutig voneinander zu differenzieren wie beim VEGF₁₆₅-Wildtyp. Im Gegensatz zum VEGF-Wildtyp zeigt das mutierte Protein bis zu 240 Minuten nach Inkubation keinerlei Veränderung im Laufverhalten.

Diese Beobachtung legt die Vermutung nahe, dass die Mutation Arginin₁₁₀ zu Alanin₁₁₀ zur Inaktivierung der Plasmin-Schnittstelle geführt hat. Wie in Figur 1B weiter dargestellt, weisen auch die drei weiteren Mutanten Mut_{Pro}, Mut_{Gln} und Mut_{Lys-Pro} nach Inkubation mit Plasmin über 240 Minuten eine vergleichbare Stabilität der Signalbanden bei 45 und 42 kDA auf. Eine Kontrolle, bei der VEGF₁₆₅-Wildtyp über 4 Stunden bei 37 °C mit Plasminpuffer inkubiert wurde, wird nicht degradiert (Bahnen 18 und 19). Insgesamt weisen diese Experimente darauf hin, dass die erzeugten und aufgereinigten VEGF-Mutanten stabil gegenüber der Protease Plasmin sind.

### b) Inkubation in akuter und chronischer Wundflüsigkeit

Im nächsten Schritt wurde die Degradation der VEGF-Mutanten in Wundflüssigkeit von Patienten mit akuten und chronischen Wunden analysiert. Bei Inkubation des VEGF₁₆₅ Wildtyps und aller VEGF-Mutanten in akuter Wundflüssigkeit war keine Degradation nach 240 Minuten nachweisbar.

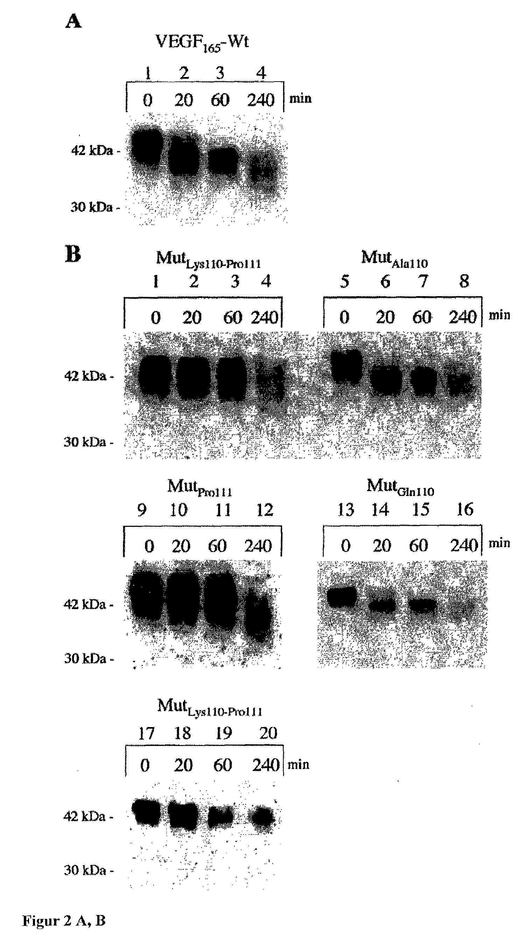

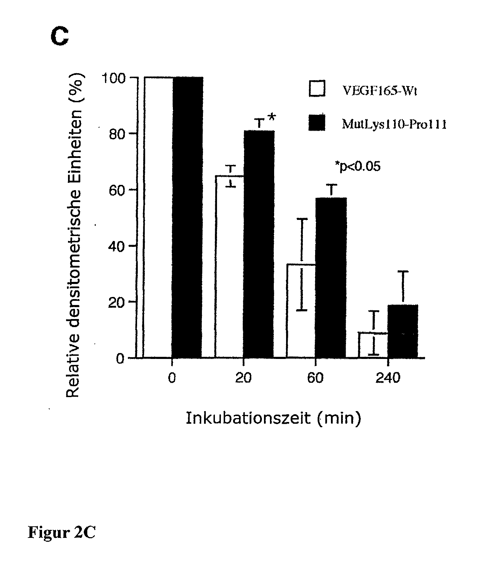

Figur 2 zeigt den Effekt chronischer Wundflüssigkeit auf die Stabilität der VEGF-Proteine. Die Inkubation des in COS-1-Zellen synthetisierten VEGF-Wildtyps (Figur 2A, Bahn 1-4) über 240 Minuten zeigt eine Degradation des Wachstumsfaktors mit einem Fragment von ca. 38 kDa. Dieses entspricht dem 110 Dimer-Fragment der weniger glykosylierten VEGF-Variante.

Im Gegensatz zum Wildtyp zeigen die VEGF₁₆₅-Mutanten ein anderes Degradationsverhalten bei der Inkubation in chronische Wundflüssigkeit. Auf der einen Seite ist in den Mutationen Mut_{Gln} (Figur 2B, Bahnen 13 - 16 und Mut_{Ala} (Bahnen 5 - 8) ein Degradationsprozeß zu beobachten, der mit dem Wildtyp vergleichbar ist. Es entstehen bereits nach 20 min Fragmente mit einem Molekulargewicht von ca. 38 kDa.

Auf der anderen Seite zeigt die Analyse der Mutanten Mut_{Pro} (Bahnen 9 - 12) und Mut_{Lys-Pro} (Bahnen 1 - 4, 17 - 20) ein vom Wildtyp und den Mutanten Mut_{Ala} und Mut_{Gln} abweichendes Abbauverhalten. Bis zu 60 Minuten nach Inkubation zeigt sich im SDS-PAGE ein stabiles Signal bei 42 und 45 kDa. Dies weist auf eine Stabilisierung der mutierten Proteine Mut_{Pro} und Mut_{Lys-Pro} in der chronischen Wundflüssigkeit hin. Dieser Unterschied im Degradationsverhalten von den Mutanten mit neutraler/unpolarer Aminosäure und denen mit Prolin legt die Vermutung nahe, dass im chronischen Wundmilieu neben Plasmin weitere Proteasen am Abbau von VEGF beteiligt sind.

240 Minuten nach der Inkubation in chronischer Wundflüssigkeit ist bei allen mutierten Proteinen eine Degradierung zu beobachten. Dabei kommt es nicht zur Bildung klar definierter Abbaufragmente, vielmehr entsteht nach 240 min ein diffuses Signal zwischen 38 und 45 kDa. Vermutlich handelt sich hierbei um Proteolyse im Bereich der ersten 20 Aminosäuren (Erkennungsstelle des Antikörpers), da die Signalstärke nach 240 min deutlich abnimmt.

Zusammenfassend deuten die Ergebnisse darauf hin, dass die VEGF-Mutanten mit Prolin an Position 111 zunächst in der chronischen Wundflüssigkeit stabilisiert sind, langfristig jedoch degradiert werden. Es wurden vergleichbare Ergebnisse in den Wundflüssigkeiten von drei verschiedenen Patienten mit chronisch venöser Insuffizienz beobachtet. Die Experimente für die verschiedenen Wundflüssigkeiten wurden mindestens zweimal wiederholt (Figur 2B: Patient X Bahnen 1 - 4; Patient Y Bahnen 17 - 20). Dabei blieb das entstehende Bandenmuster stets gleich.

In Figur 2C ist eine densitometrische Auswertung des Abbaus von VEGF-Wildtyp und Mut_{Lys-Pro} dargestellt. Ziel der Untersuchung war die Quantifizierung der Stabilisierung der VEGF-Mutante in der chronischen Wundflüssigkeit. Zu diesem Zweck wurde die zeitabhängige Veränderung der Signalstärke in der Höhe des Ausgangssignals (Bereich 42-45 kDa) im Vergleich zum Signal am Zeitpunkt Null bestimmt. Die zu den unterschiedlichen Zeitpunkten gemessenen densitometrischen Dichten sind als Prozent des Ausgangssignals dargestellt. In dieser densitometrischen Untersuchung wird deutlich, dass zu jedem Zeitpunkt der Messung die VEGF-Mutante im Vergleich zum VEGF-Wildtyp ein stärkeres Signal im Bereich 42-45 kDa zeigt und somit intaktes VEGF₁₆₅-Protein vorliegt. Diese Beobachtung legt die Vermutung nahe, dass diese Mutation zu einer verbesserten Stabilität und Bioaktivität des VEGF-Proteins im chronischen Wundmilieu führt. Bereits 20 Minuten nach der Inkubation ist der Unterschied zwischen Wildtyp und Mutante statistisch signifikant. Die Messungen wurden für drei unabhängige Experimente mit identischer Wundflüssigkeit durchgeführt.

### II. Untersuchungen zur biologischen Aktivität von VEGF₁₆₅-Wildtyp und den mutierten Varianten:

Es wurde untersucht, ob die Mutationen einen Einfluß auf die biologische Aktivität des VEGF-Moleküls haben. Die biologische Aktivität wurde mittels eines BrdU-Proliferations-Assays (Roche Diagnostics, Mannheim) auf Human Umbilical Vein Endothelial Cells (HUVE-Zellen) nach Angaben des Herstellers getestet. Dabei wurden die HUVE-Zellen unter Zugabe unterschiedlicher VEGF-Mutanten kultiviert, danach 6 Stunden mit BrdU-Lösung inkubiert und fixiert, wonach ein ELISA unter Verwendung eines BrdU-spezifischen Antikörpers durchgeführt wurde.

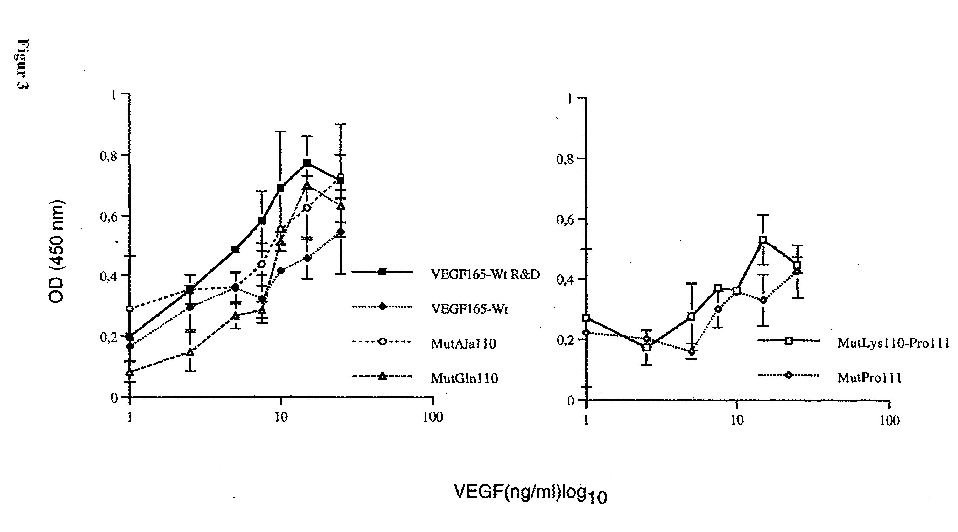

Es kamen VEGF-Konzentrationen zwischen 1 ng/ml und 25 ng/ml zum Einsatz. Kommerziell erhältliches rekombinantes VEGF₁₆₅-Protein (R&D Sytems) und in COS-1-Zellen synthetisierter VEGF₁₆₅-Wildtyp zeigte eine halb-maximale Stimulation der BrdU-Inkorporation bei ca. 3 ng/ml (Figur 3). Die mutierten VEGF-Proteine sind durch eine der in COS-1-Zellen synthetisierten VEGF-Wildtyp vergleichbare Stimulation der Endothelzellenproliferation charakterisiert. Die maximale Stimulation aller in COS-1-Zellen synthetisierten Proteine war geringer als die durch kommerziell erhältlichen VEGF₁₆₅-Wildtyp. Der Unterschied zwischen den beiden Kurvenverläufen kann durch die unterschiedlichen Expressionssysteme und Aufreinigungsmethoden der Proteine begründet werden (Mohanraj *et al.* 1995). Die biologische Aktivität von VEGF₁₆₅ wird also durch die durchgeführten Mutationen nicht signifikant beeinflusst.

Im folgenden wurde der Frage nachgegangen, inwieweit die biologische Aktivität des VEGF₁₆₅-Wildtyps und der VEGF-Mutanten nach Plasmininkubation beeinflusst wird. Zu diesem Zweck wurden die VEGF-Proteine mit Plasmin inkubiert und anschließend die biologische Aktivität mittels eines BrdU-Proliferationsassay auf HUVE-Zellen untersucht.

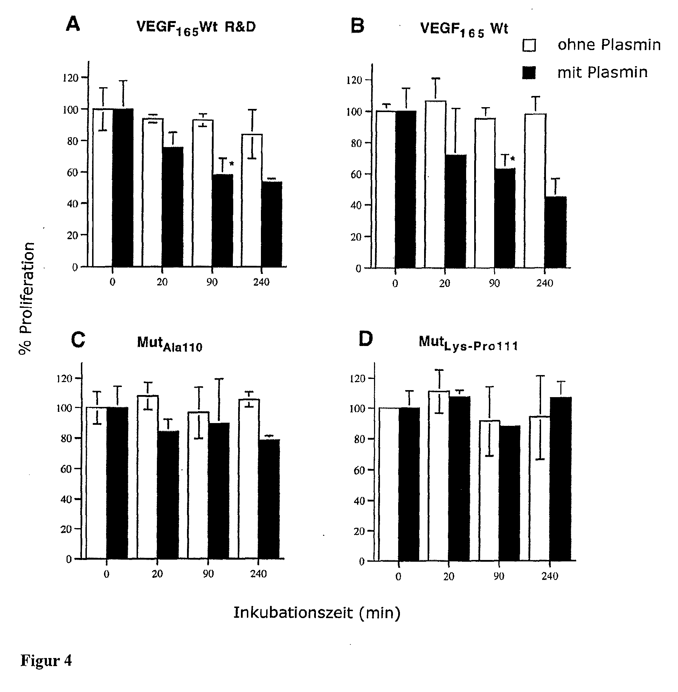

In der graphischen Darstellung (Figur 4) ist die BrdU-Inkorporation als Prozent des Ausgangssignal (Zeitpunkt t = 0) dargestellt. Die Inkubation der VEGF-Wildtypen (in COS-1-Zellen synthetisiert und von R&D Systems) sowie der VEGF-Mutanten Mut_{Ala} und Mut_{Lys-Pro} in Plasminpuffer bei 37 °C zeigt keine Beeinträchtigung der biologische Aktivität der Proteine (Figur 4 A-D). Im Gegensatz dazu führt die Inkubation der VEGF₁₆₅-Wildtypen in Plasmin zu einer deutlichen Verringerung der biologischen Aktivität (Figur 4 A, B). Schon 20 Minuten nach Inkubation zeigt sich ein Aktivitätsverlust von mindestens 20%, der dann bis auf ca. 50% der Ausgangaktivität nach 240 Minuten weiter abfällt. Die Mutanten Mut_{Ala} und Mut_{Lys-Pro} zeigen keinen signifikanten Aktivitätsverlust nach Inkubation mit Plasmin (Figur 4 C, D). Diese Ergebnisse unterstreichen die im Western-Blot dargestellte "Plasmin-Resistenz" der Mutanten (Figur 1) und zeigen, dass die mutierten Proteine auch nach Inkubation mit Plasmin stabil sind.

Die eingefügten Mutationen haben also eine Hemmung der VEGF-Spaltung durch Plasmin zur Folge. Durch die Mutation von Ala₁₁₁ zu Pro₁₁₁ kann eine Stabilisierung von VEGF und damit eine erhöhten biologischen Aktivität im chronischen Wundmilieu herbeigeführt werden.
**Figur 1:** Die VEGF₁₆₅-Mutationen sind resistent gegenüber der Spaltung durch Plasmin. Die Figur zeigt die Inkubation von VEGF₁₆₅ und den mutierten Proteinen in einer Plasminlösung [0,01 U/ml] oder Pufferlösung (B, Bahnen 18, 19) für die angegebenen Zeiträume. Die Analyse des Degradationsverhaltens erfolgte durch Western-Blot und Immundetektion.
**Figur 2:** Die Mutation von Ala₁₁₁ zu Pro₁₁₁ erhöht die Stabilität von VEGF in chronischer Wundflüssigkeit. A) In COS-1-Zellen exprimierter VEGF₁₆₅-Wildtyp und B) die VEGF-Varianten wurden für die angegebenen Zeiträume in chronischer Wundflüssigkeit inkubiert und das Degradationsverhalten durch Immundetektion dargestellt. Dabei wurden Wundflüssigkeiten von zwei unterschiedlichen Patienten untersucht: Patient X, Bahnen 1 - 16; Patient Y: Bahnen 17 - 20). C) Densitometrische Darstellung der Degradation von VEGF-Wildtyp und Mut_{Lys-Pro} in chronischer Wundflüssigkeit. Dargestellt ist die relative Signalstärke aus drei unabhängig durchgeführten Western-Blot-Analysen (MW +/- SD).
**Figur 3:** Die VEGF-Mutanten sind biologisch aktiv. VEGF₁₆₅-Wildtyp und VEGF-Mutanten wurden jeweils in steigenden Konzentrationen mit HUVE-Zellen inkubiert. Dargestellt ist die durch BrdU-Elisa bestimmte Einbaurate des Basen-Analogons in die DNA der proliferierenden Zellen (MW +/- SD; n=3).
**Figur 4 :** Plasmin verändert nicht die biologische Aktivität der VEGF₁₆₅-Mutanten. Gegenübergestellt ist die relative BrdU Inkorporation in HUVE-Zellen durch Stimulation mit VEGF₁₆₅-Wildtyp (A, B), Mut_{Ala} (C) und Mut_{Lys-Pro} (D) nach Inkubation des angegebenen Proteins in Puffer oder Plasmin (Mittelwerte +/-SD; n = 3).

### Referenzen:

Frank S, Hübner G, Breier G, *et al*., J Biol Chem 270:12607-12613, 1995.
Fukumura D, Xavier R, Sugiura T, *et al*., Cell 94:715-725, 1998.
Gospodarowicz D, Abraham J, Schilling J, Proc Natl Acad Sci 86:7311-7315, 1989.
Keck P, Hauser S, Krivi G, *et al*., Science 246:1309-1312, 1989.
Keyt B, Berleau L, Ngyen H, Chen H *et al*., J Biol Chem 271:7788-7795, 1996.
Lauer G, Sollberg S, Cole M, Flamme I, Stürzebecher J, Mann K, Krieg T, Eming S, J Invest Dermatol 115:12-18, 2000.
Lawrence W, Diegelann R, Clin Dermatol 12:157-169, 1994.
Mohanraj D, Olson T, Ramakrishnan S, *et al*., Growth Factors 12:17-27, 1995.
Nissen N, Polverini P, Koch A, *et al*., Am J Path 152:1445-1452, 1998.
Poers J, Odake S, Oleksyszyn J, Hori H *et al*., AAS 42:3-18, 1993.
Scharffetter-Kochanek, Meewes C, Eming S, *et al*., Z. Hautkrankheiten 74:239-249, 1999.
Soker S, Takashima S, Miao H, Neufeld G, Klagsbrun M *et al*., Cell 92:735-745, 1998.
Stryer T, in Biochemie. 4. Auflage 1987; Spektrum Akademischer Verlag.

## Patentansprüche

1. Vascular Endothelial Growth Factor (VEGF)-Variante, dadurch gekennzeichntet, dass das Alanin an AS-Position 111 gegen Prolin ausgetauscht ist.

2. VEGF-Variante nach Anspruch 1, **dadurch gekennzeichnet, dass** das Arginin an AS-Position 110 gegen eine andere Aminosäure ausgetauscht ist.

3. VEGF-Variante nach Anspruch 1 und/oder 2, wobei die VEGF-Variante als eine der Splice-Varianten VEGF₁₂₁, VEGF₁₄₅, VEGF₁₆₅, VEGF₁₈₃, VEGF₁₈₉ oder VEGF₂₀₆ vorliegt.

4. VEGF₁₆₅ -Variante nach einem der Ansprüche 1 bis 3, **dadurch gekennzeichnet, dass** sie eine der Aminosäuresequenzen 
Seq. No. 1:
Ala Pro Met Ala Glu Gly Gly Gly Gln Asn His His Glu Val Val
Lys Phe Met Asp Val Tyr Gln Arg Ser Tyr Cys His Pro Ile Glu
Thr Leu Val Asp Ile Phe Gln Glu Tyr Pro Asp Glu Ile Glu Tyr
Ile Phe Lys Pro Ser Cys Val Pro Leu Met Arg Cys Gly Gly Cys
Cys Asn Asp Glu Gly Leu Glu Cys Val Pro Thr Glu Glu Ser Asn
Ile Thr Met Gln Ile Met Arg Ile Lys Pro His Gln Gly Gln His
Ile Gly Glu Met Ser Phe Leu Gln His Asn Lys Cys Glu Cys Arg
Pro Lys Lys Asp Arg Pro Arg Gln Glu Asn Pro Cys Gly Pro Cys
Ser Glu Arg Arg Lys His Leu Phe Val Gln Asp Pro Gln Thr Cys
Lys Cys Ser Cys Lys Asn Thr Asp Ser Arg Cys Lys Ala Arg Gln
Leu Glu Leu Asn Glu Arg Thr Cys Arg Cys Asp Lys Pro Arg Arg
 
oder Seq. No. 2:

Ala Pro Met Ala Glu Gly Gly Gly Gln Asn His His Glu Val Val
Lys Phe Met Asp Val Tyr Gln Arg Ser Tyr Cys His Pro Ile Glu
Thr Leu Val Asp Ile Phe Gln Glu Tyr Pro Asp Glu Ile Glu Tyr
Ile Phe Lys Pro Ser Cys Val Pro Leu Met Arg Cys Gly Gly Cys
Cys Asn Asp Glu Gly Leu Glu Cys Val Pro Thr Glu Glu Ser Asn
 
Ile Thr Met Gln Ile Met Arg Ile Lys Pro His Gln Gly Gln His
Ile Gly Glu Met Ser Phe Leu Gln His Asn Lys Cys Glu Cys Arg
Pro Lys Lys Asp Lys Pro Arg Gln Glu Asn Pro Cys Gly Pro Cys
Ser Glu Arg Arg Lys His Leu Phe Val Gln Asp Pro Gln Thr Cys
Lys Cys Ser Cys Lys Asn Thr Asp Ser Arg Cys Lys Ala Arg Gln
Leu Glu Leu Asn Glu Arg Thr Cys Arg Cys Asp Lys Pro Arg Arg
 aufweist.

5. VEGF-Variante nach einem der Ansprüche 1 bis 4, **dadurch gekennzeichnet**, das die Aminosäurekette modifiziert oder derivatisiert ist und/oder Mutationen, Insertionen und/oder Deletionen enthält, und/oder dass sie eine Signalsequenz aufweist.

6. VEGF-Variante nach Anspruch 5, **dadurch gekennzeichnet, dass** die Signalsequenz N-terminal an die Aminosäurekette der VEGF-Variante anschließt und die Sequenz 
Met Asn Phe Leu Ser Trp Ser Val His Trp Ser Leu Ala Leu
Leu Leu Tyr Leu His His Ala Lys Trp Ser Gln Ala
 aufweist.

7. Nucleinsäuren, kodierend für VEGF-Varianten nach einem der Ansprüche 1
bis 6.

8. Vektoren, enthaltend Nucleinsäuren nach Anspruch 7 zur Expression von VEGF-Varianten nach einem der Ansprüche 1 bis 6.

9. Arzneimittel, enthaltend VEGF-Varianten nach einem der Ansprüche 1 bis 6, Nucleinsäuren nach Anspruch 7 oder Vektoren nach Anspruch 8.

10. Verwendung von VEGF-Varianten nach einem der Ansprüche 1 bis 6, von Nucleinsäuren nach Anspruch 7 oder von Vektoren nach Anspruch 8 zur Herstellung eines Arzneimittels zur Behandlung von chronischen Wunden, insbesondere verursacht durch Gefäßveränderungen wie chronisch-venöse Insuffizienz (CVI), primäres/sekundäres Lymphödem, arterielle Verschlußkrankheit, metabolische Erkrankungen wie Diabetes mellitus, Gicht oder Dekubitus, chronisch entzündlichen Erkrankungen wie Pyoderma gangraenosum, Vaskulitis, perforierende Dermatosen wie Necrobiosis lipoidica diabeticorum und Granuloma annulare, hämatologische Grunderkrankungen wie Gerinnungsstörungen, Sichelzellenanämie und Polycythemia vera, Tumoren wie primäre kutane Tumoren und exulzerierte Metastasen, zur Plasmininhibition, zur Induktion der Neoangiogenese und/oder zur Inhibition der Matrixdegradierung.


SEQUENZPROTOKOLL

<110> Eming, Sabine A.

<120> Proteolyseresistenter aktiver VEGF

<130> 012767ep

<140>
<141>

<160> 3

<170> PatentIn Ver. 2.1

<210> 1
<211> 165
<212> PRT
<213> Künstliche Sequenz

<220>
<223> Beschreibung der künstlichen Sequenz: mutierter
      humaner VEGF

<400> 1
Ala Pro Met Ala Glu Gly Gly Gly Gln Asn His His Glu Val Val Lys
  1               5                  10                  15

Phe Met Asp Val Tyr Gln Arg Ser Tyr Cys His Pro Ile Glu Thr Leu
             20                  25                  30

Val Asp Ile Phe Gln Glu Tyr Pro Asp Glu Ile Glu Tyr Ile Phe Lys
         35                  40                  45

Pro Ser Cys Val Pro Leu Met Arg Cys Gly Gly Cys Cys Asn Asp Glu
     50                  55                  60

Gly Leu Glu Cys Val Pro Thr Glu Glu Ser Asn Ile Thr Met Gln Ile
 65                  70                  75                  80

Met Arg Ile Lys Pro His Gln Gly Gln His Ile Gly Glu Met Ser Phe
                 85                  90                  95

Leu Gln His Asn Lys Cys Glu Cys Arg Pro Lys Lys Asp Arg Pro Arg
            100                 105                 110

Gln Glu Asn Pro Cys Gly Pro Cys Ser Glu Arg Arg Lys His Leu Phe
        115                 120                 125

Val Gln Asp Pro Gln Thr Cys Lys Cys Ser Cys Lys Asn Thr Asp Ser
    130                 135                 140

Arg Cys Lys Ala Arg Gln Leu Glu Leu Asn Glu Arg Thr Cys Arg Cys
145                 150                 155                 160

Asp Lys Pro Arg Arg
                165

<210> 2
<211> 165
<212> PRT
<213> Künstliche Sequenz

<220>
<223> Beschreibung der künstlichen Sequenz: mutierter
      humaner VEGF

<400> 2
Ala Pro Met Ala Glu Gly Gly Gly Gln Asn His His Glu Val Val Lys
  1               5                  10                  15

Phe Met Asp Val Tyr Gln Arg Ser Tyr Cys His Pro Ile Glu Thr Leu
             20                  25                  30

Val Asp Ile Phe Gln Glu Tyr Pro Asp Glu Ile Glu Tyr Ile Phe Lys
         35                  40                  45

Pro Ser Cys Val Pro Leu Met Arg Cys Gly Gly Cys Cys Asn Asp Glu
    50                  55                  60

Gly Leu Glu Cys Val Pro Thr Glu Glu Ser Asn Ile Thr Met Gln Ile
 65                  70                  75                  80

Met Arg Ile Lys Pro His Gln Gly Gln His Ile Gly Glu Met Ser Phe
                 85                  90                  95

Leu Gln His Asn Lys Cys Glu Cys Arg Pro Lys Lys Asp Lys Pro Arg
            100                 105                 110

Gln Glu Asn Pro Cys Gly Pro Cys Ser Glu Arg Arg Lys His Leu Phe
        115                 120                 125

Val Gln Asp Pro Gln Thr Cys Lys Cys Ser Cys Lys Asn Thr Asp Ser
    130                 135                 140

Arg Cys Lys Ala Arg Gln Leu Glu Leu Asn Glu Arg Thr Cys Arg Cys
145                 150                 155                 160

Asp Lys Pro Arg Arg
                165

<210> 3
<211> 26
<212> PRT
<213> Homo sapiens

<400> 3
Met Asn Phe Leu Ser Trp Ser Val His Trp Ser Leu Ala Leu Leu Leu
  1               5                  10                  15

Tyr Leu His His Ala Lys Trp Ser Gln Ala
                20                  25